# EUROPEAN PATENT APPLICATION

(11) **EP 3 940 073 A1**
(43) Date of publication of application: **19.01.2022**
(21) Application number: 20770225.9
(22) Date of filing: 10.03.2020
(51) Int. Cl.: C12N 15/09, C12N 15/10, C12Q 1/6848, C12Q 1/6869

(54) **PRODUCTION AND AMPLIFICATION OF NUCLEIC ACIDS**

(30) Priority: 13.03.2019 JP 2019046275
(71) Applicant: TOYOBO CO., LTD., Osaka-shi Osaka 530-8230 (JP); RIKEN, Wako-shi Saitama 351-0198 (JP)
(72) Inventor: YAMAKOSHI, Nana, Tsuruga-shi, Fukui 914-8550 (JP); HAYASHI, Tetsutaro, Wako-shi, Saitama 351-0198 (JP); NIKAIDO, Itoshi, Wako-shi, Saitama 351-0198 (JP)
(74) Representative: Vossius & Partner Patentanwälte Rechtsanwälte mbB
(86) International application number: PCT/JP2020/010248
(87) International publication number: WO 2020/184551

(57) **Abstract**

Provided is a composition and method for suppressing the reverse transcription of ribosomal RNA. The composition for suppressing the reverse transcription of ribosomal RNA contains at least one nucleic acid polymer selected from the group consisting of inosinic acid polymers, cytidylic acid polymers, guanylic acid polymers, adenylic acid polymers, thymidylic acid polymers, uridylic acid polymers, deoxyinosinic acid polymers, deoxycytidylic acid polymers, deoxyguanylic acid polymers, deoxyadenylic acid polymers, deoxythymidylic acid polymers, and deoxyuridylic acid polymers.

SEQUENCE LISTING

<110>  TOYOBO CO., LTD.
       RIKEN

<120>  Genaration and amplification of nucleic acids

<130>  P19-248WO

<150>  JP 2019-046275
<151>  2019-03-13

<160>  8

<170>  PatentIn version 3.5

<210>  1
<211>  20
<212>  DNA
<213>  Artificial Sequence

<220>
<223>  Primer

<400>  1
cagctgagag ggaaatcgtg                                                   20


<210>  2
<211>  20
<212>  DNA
<213>  Artificial Sequence

<220>
<223>  Primer

<400>  2
cgttgccaat agtgatgacc                                                   20


<210>  3
<211>  20
<212>  DNA
<213>  Artificial Sequence

<220>
<223>  Primer

<400>  3
gatcgggacc ccagtgacct                                                   20


<210>  4
<211>  20
<212>  DNA
<213>  Artificial Sequence

<220>
<223>  Primer

<400>  4
agctttcccc aacacccaca                                                   20

<210>  5
<211>  21
<212>  DNA
<213>  Artificial Sequence

<220>
<223>  Primer

<400>  5
tcagtcaacg ggggacataa a                                                21


<210>  6
<211>  21
<212>  DNA
<213>  Artificial Sequence

<220>
<223>  Primer

<400>  6
ggggctgtac tgcttaacca g                                                21


<210>  7
<211>  20
<212>  DNA
<213>  Artificial Sequence

<220>
<223>  Primer

<400>  7
ctcaacacgg gaaacctcac                                                  20


<210>  8
<211>  20
<212>  DNA
<213>  Artificial Sequence

<220>
<223>  Primer

<400>  8
cgctccacca actaagaacg                                                  20

## Description

### Technical Field

The present invention relates to the production and amplification of nucleic acids. The present invention relates to, for example, a composition and method useful in producing and amplifying a nucleic acid from a ribonucleic acid (RNA) template, specifically, a composition and method for producing and amplifying a nucleic acid by reverse transcription reaction by using a specific nucleic acid polymer. The composition and the method according to the present invention are used in, for example, suppressing the reverse transcription of ribosomal RNA, more specifically, suppressing the synthesis of DNA derived from ribosomal RNA.

### Background Art

Ribosomal RNA is thought to account for about 80 to 90% of RNA in vivo. In the analysis of gene expression levels, it is common to convert expressed RNA into cDNA by reverse transcription reaction and perform gene analysis based on the cDNA. If ribosomal RNA, which makes up the majority of RNA in vivo, is reverse-transcribed and amplified during this reverse transcription reaction, noise is generated in the expression analysis of RNA that should be reverse-transcribed and amplified. Additionally, in the recent years, new technologies, such as an RT-RamDA method, that enable the generation of a large amount of amplified nucleic acid products by using RNA as a template, have been developed (see PTL 1). In these techniques such as the RT-RamDA method capable of generating a substantial amount of amplified nucleic acid products using RNA as a template, once the reverse transcription reaction of ribosomal RNA occurs, ribosomal RNA is isothermally amplified by strand displacement activity. This greatly affects the analysis, reducing the relative amount of RNA that should be analyzed. Thus, it is desirable to suppress the synthesis of DNA derived from ribosomal RNA in nucleic acid analysis involving reverse transcription reaction.

### Citation List

### Patent Literature

PTL 1: WO2016/052619A

### Summary of Invention

### Technical Problem

An object is to provide a means for suppressing the reverse transcription of ribosomal RNA.

### Solution to Problem

The present inventors conducted extensive research to achieve the object and found that the use of a specific nucleic acid polymer can suppress the reverse transcription of ribosomal RNA and suppress the synthesis of DNA derived from ribosomal RNA in a method of synthesizing DNA by reverse transcription of template RNA. The inventors conducted further research based on this finding and completed the present invention.

Specifically, the present invention includes the following subject matter.

### Item 1.

A method for synthesizing DNA by reverse transcription of template RNA, the method comprising suppressing synthesis of DNA derived from ribosomal RNA using at least one nucleic acid polymer selected from the group consisting of inosinic acid polymers, cytidylic acid polymers, guanylic acid polymers, adenylic acid polymers, thymidylic acid polymers, uridylic acid polymers, deoxyinosinic acid polymers, deoxycytidylic acid polymers, deoxyguanylic acid polymers, deoxyadenylic acid polymers, deoxythymidylic acid polymers, and deoxyuridylic acid polymers.

### Item 2.

The method according to Item 1, wherein the nucleic acid polymer is at least one homopolymer selected from the group consisting of polyinosinic acid, polycytidylic acid, polyguanylic acid, polyadenylic acid, polythymidylic acid, polyuridylic acid, polydeoxyinosinic acid, polydeoxycytidylic acid, polydeoxyguanylic acid, polydeoxyadenylic acid, polydeoxythymidylic acid, polydeoxyuridylic acid, and salts thereof.

### Item 3.

The method according to Item 1 or 2, wherein the nucleic acid polymer is at least one homopolymer selected from the group consisting of polyinosinic acid, polycytidylic acid, polyguanylic acid, polydeoxyinosinic acid, polydeoxycytidylic acid, polydeoxyguanylic acid, and salts thereof.

### Item 4.

The method according to any one of Items 1 to 3, wherein the nucleic acid polymer is at least one homopolymer selected from the group consisting of polyinosinic acid, polydeoxyinosinic acid, and salts thereof.

### Item 5.

The method according to any one of Items 1 to 4, wherein the nucleic acid polymer has a total length of 30 to 10000 bases.

### Item 6.

The method according to any one of Items 1 to 5, wherein the reverse transcription is performed by using a random primer.

### Item 7.

The method according to Item 6, wherein the random primer has a base sequence that is not completely complementary to the base sequence of the ribosomal RNA.

### Item 8.

The method according to any one of Items 1 to 7, wherein the reverse transcription is performed in an RT-PCR method or RT-RamDA method.

### Item 9.

A composition for suppressing reverse transcription of ribosomal RNA (or suppressing synthesis of DNA derived from ribosomal RNA in synthesis of DNA by reverse transcription of template RNA), the composition comprising at least one nucleic acid polymer selected from the group consisting of inosinic acid polymers, cytidylic acid polymers, guanylic acid polymers, adenylic acid polymers, thymidylic acid polymers, uridylic acid polymers, deoxyinosinic acid polymers, deoxycytidylic acid polymers, deoxyguanylic acid polymers, deoxyadenylic acid polymers, deoxythymidylic acid polymers, and deoxyuridylic acid polymers.

### Item 9A.

Use of a nucleic acid polymer in suppressing reverse transcription of ribosomal RNA (or suppressing synthesis of DNA derived from ribosomal RNA in synthesis of DNA by reverse transcription of template RNA), wherein the nucleic acid polymer is at least one nucleic acid polymer selected from the group consisting of inosinic acid polymers, cytidylic acid polymers, guanylic acid polymers, adenylic acid polymers, thymidylic acid polymers, uridylic acid polymers, deoxyinosinic acid polymers, deoxycytidylic acid polymers, deoxyguanylic acid polymers, deoxyadenylic acid polymers, deoxythymidylic acid polymers, and deoxyuridylic acid polymers.

### Item 10.

A reverse transcription reaction composition for preparing a reverse transcription reaction product for use in next-generation sequencing, the reverse transcription reaction composition comprising at least one nucleic acid polymer selected from the group consisting of inosinic acid polymers, cytidylic acid polymers, guanylic acid polymers, adenylic acid polymers, thymidylic acid polymers, uridylic acid polymers, deoxyinosinic acid polymers, deoxycytidylic acid polymers, deoxyguanylic acid polymers, deoxyadenylic acid polymers, deoxythymidylic acid polymers, and deoxyuridylic acid polymers.

### Item 11.

The composition according to Item 9 or 10 or the use according to Item 9A, wherein the reverse transcription is performed in an RT-PCR method or RT-RamDA method.

### Item 12.

The method according to any one of Items 1 to 8, the composition according to any one of Items 9 to 11, or the use according to Item 9A, wherein the nucleic acid polymer contains a structural unit derived from a nucleotide in an amount of 60 mol% or more of the total structural units; and wherein the nucleotide is selected from the group consisting of inosinic acid, cytidylic acid, guanylic acid, deoxyinosinic acid, deoxycytidylic acid, deoxyguanylic acid, derivatives thereof, and salts thereof.

### Item 13.

The method according to any one of Items 1 to 8 and 12, the composition according to any one of Items 9 to 12, or the use according to Item 9A, wherein the nucleic acid polymer contains a structural unit derived from a nucleotide in an amount of 90 mol% or more of the total structural units; and wherein the nucleotide is selected from the group consisting of inosinic acid, cytidylic acid, guanylic acid, deoxyinosinic acid, deoxycytidylic acid, deoxyguanylic acid, derivatives thereof, and salts thereof.

### Item 14.

The method according to any one of Items 1 to 8, 12, and 13, the composition according to any one of Items 9 to 13, or the use according to Item 9A, wherein the nucleic acid polymer contains a structural unit derived from a nucleotide in an amount of 60 mol% or more of the total structural units; and wherein the nucleotide is selected from the group consisting of inosinic acid, deoxyinosinic acid, derivatives thereof, and salts thereof.

### Item 15.

The method according to any one of Items 1 to 8 and 12 to 14, the composition according to any one of Items 9 to 14, or the use according to Item 9A, wherein the nucleic acid polymer contains a structural unit derived from a nucleotide in an amount of 90 mol% or more of the total structural units; and wherein the nucleotide is selected from the group consisting of inosinic acid, deoxyinosinic acid, derivatives thereof, and salts thereof.

### Item 16.

The method according to any one of Items 1 to 8 and 12 to 15, wherein the template RNA is RNA extracted from a cell.

### Item 17.

A method for producing DNA comprising performing reverse transcription of template RNA in the presence of a nucleic acid polymer (which is not a primer), wherein the nucleic acid polymer is at least one member selected from the group consisting of inosinic acid polymers, cytidylic acid polymers, guanylic acid polymers, adenylic acid polymers, thymidylic acid polymers, uridylic acid polymers, deoxyinosinic acid polymers, deoxycytidylic acid polymers, deoxyguanylic acid polymers, deoxyadenylic acid polymers, deoxythymidylic acid polymers, and deoxyuridylic acid polymers.

### Item 18.

The method according to Item 17, wherein the template RNA is RNA extracted from a cell.

### Item 19.

A composition for reverse transcription comprising an inosinic acid polymer (which excludes a composition for reverse transcription comprising guanidine thiocyanate).

### Item 20.

The composition according to Item 19, wherein the concentration of the inosinic acid polymer is less than 1 ng/uL.

### Item 21.

The composition according to Item 19 or 20, which is for use in an RT-PCR method or RT-RamDA method.

### Item 22.

The composition according to any one of Items 9 to 15 and 19 to 21, which is in the form of a kit.

### Advantageous Effects of Invention

By the use of the nucleic acid polymer according to the present invention, the reverse transcription of ribosomal RNA or the synthesis of DNA derived from ribosomal RNA can be suppressed.

### Brief Description of Drawings

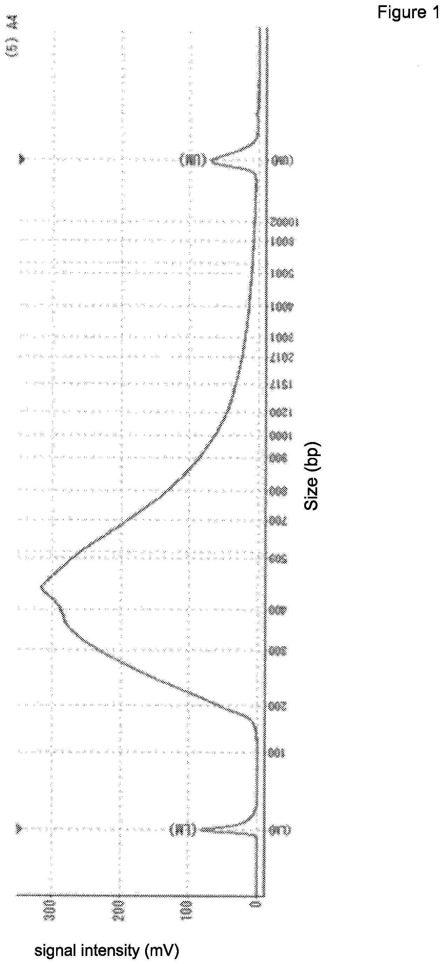

Fig. 1 illustrates the results (reference value) of the measurement of base length distribution of the polyinosinic acid potassium salt used in Examples 1 to 4 by using a DNA-12000 reagent kit (Shimadzu Corporation).

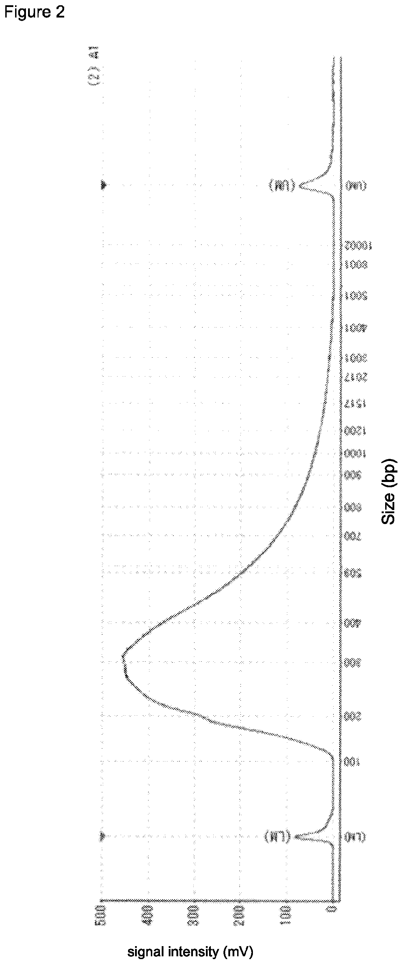

Fig. 2 illustrates the results (reference value) of the measurement of base length distribution of the polyadenylic acid potassium salt used in Example 4 by using a DNA-12000 reagent kit (Shimadzu Corporation).

### Description of Embodiments

### A method for synthesizing DNA by reverse transcription of template RNA, the method comprising suppressing the synthesis of DNA derived from ribosomal RNA (rRNA) by using a nucleic acid polymer

### 1. Nucleic Acid Polymer

The nucleic acid polymer is preferably at least one member selected from the group consisting of inosinic acid polymers, cytidylic acid polymers, guanylic acid polymers, adenylic acid polymers, thymidylic acid polymers, uridylic acid polymers, deoxyinosinic acid polymers, deoxycytidylic acid polymers, deoxyguanylic acid polymers, deoxyadenylic acid polymers, deoxythymidylic acid polymers, and deoxyuridylic acid polymers. Of these, from the standpoint of more effectively suppressing the reverse transcription of rRNA or the synthesis of DNA derived from rRNA, at least one member selected from the group consisting of inosinic acid polymers, cytidylic acid polymers, guanylic acid polymers, deoxyinosinic acid polymers, deoxycytidylic acid polymers, and deoxyguanylic acid polymers is more preferable, and inosinic acid polymers and/or deoxyinosinic acid polymers are particularly preferable. In the present invention, these nucleic acid polymers may be used in a combination of two or more. For example, a combination of an inosinic acid polymer and a cytidylic acid polymer may be used, or a combination of a deoxyinosinic acid polymer and a cytidylic acid polymer may be used. When two or more nucleic acid polymers are used in combination, one of the nucleic acid polymers is preferably an inosinic acid polymer or a deoxyinosinic acid polymer.
The following describes each polymer in detail.

### (1) Inosinic Acid Polymer

The inosinic acid polymer as used here includes the meanings of an inosinic acid homopolymer (polyinosinic acid), an inosinic acid copolymer, derivatives thereof, and salts thereof. A double-stranded nucleic acid polymer referred to as, for example, "poly(I:C)" formed by annealing an inosinic acid homopolymer (polyinosinic acid) and a cytidylic acid homopolymer (polycytidylic acid) is known in the art. Like this double-stranded nucleic acid polymer, a double-stranded nucleic acid polymer of which at least one strand is an inosinic acid polymer (e.g., polyinosinic acid) is also included in the inosinic acid polymer in the present invention.

The inosinic acid copolymer is a polymer having a structural unit derived from inosinic acid (IMP) and a structural unit derived from a nucleotide other than IMP.

The structural unit derived from a nucleotide such as IMP refers to a unit composed of a nucleotide as represented by the following formula: 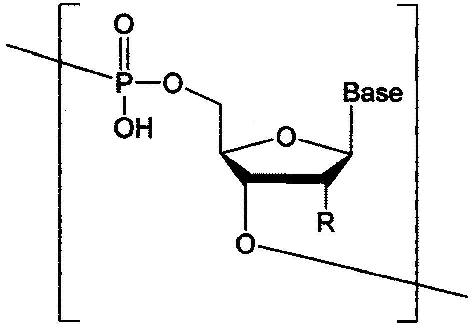 wherein R represents a hydrogen atom or hydroxyl, and the base is a nucleic acid base such as inosine, cytosine, guanine, adenine, thymine, or uracil.

The nucleotide other than IMP cannot be limited, and may be a ribonucleotide or a deoxyribonucleotide. Examples of nucleotides other than IMP include deoxyinosinic acid (dIMP), cytidylic acid (CMP), deoxycytidylic acid (dCMP), guanylic acid (GMP), deoxyguanylic acid (dGMP), adenylic acid (AMP), deoxyadenylic acid (dAMP), thymidylic acid (TMP), deoxythymidylic acid (dTMP), uridylic acid (UMP), deoxyuridylic acid (dUMP), and a combination of two or more thereof. Of these, at least one member selected from the group consisting of dIMP, CMP, dCMP, GMP, dGMP, AMP, and dAMP is preferable. At least one member selected from the group consisting of dIMP, CMP, dCMP, GMP, and dGMP is more preferable. dIMP is more preferable.

The term "derivative" refers to a polymer that is partly modified in structure while maintaining the original backbone. Examples of modification include oxidation, reduction, replacement of an atom, and introduction of a functional group. The derivative is preferably a polymer in which a functional group (e.g., a fluorine atom, a bromine atom, an iodine atom, a lower alkyl group (e.g., a C₁₋₆ alkyl group such as a methyl group and an ethyl group), an amino group, a mercapto group, or a combination of two or more thereof) is introduced in the base moiety and/or the phosphoric acid moiety is replaced by a thiophosphoric acid moiety in at least one part of the structural unit of the original polymer.
The salt is preferably an alkali metal salt, and more preferably a sodium salt and a potassium salt.

In the inosinic acid polymer, the structural unit derived from IMP is present in an amount of, for example, over 50 mol%, preferably 60 mol% or more, more preferably 65 mol% or more, more preferably 70 mol% or more, more preferably 75 mol% or more, more preferably 80 mol% or more, more preferably 85 mol% or more, more preferably 90 mol% or more, or more preferably 95 mol% or more in the total structural units. The structural unit derived from IMP may be present in an amount of 100 mol% (i.e., a homopolymer), or for example, 99.9 mol% or less, or 99 mol% or less in the total structural units.

In the inosinic acid polymer, the number of repetitions of the structural unit derived from IMP (this can also be expressed as, for example, a base length) may be, for example 30 or more, preferably 40 or more, more preferably 50 or more, still more preferably 60 or more, yet more preferably 70 or more, for example, 100 or more, or 150 or more. The number of repetitions of the structural unit derived from IMP can also be, for example, 10000 or less, 8000 or less, 5000 or less, 3000 or less, or 1000 or less. The repetition may be continuous or intermittent, and preferably continuous.

### (2) Cytidylic Acid Polymer

The cytidylic acid polymer as used here includes the meanings of a cytidylic acid homopolymer (polycytidylic acid), a cytidylic acid copolymer, derivatives thereof, and salts thereof. A double-stranded nucleic acid polymer referred to as, for example, "poly(I:C)" formed by annealing an inosinic acid homopolymer (polyinosinic acid) and a cytidylic acid homopolymer (polycytidylic acid) is known in the art. Like this double-stranded nucleic acid polymer, a double-stranded nucleic acid polymer of which at least one strand is a cytidylic acid polymer (e.g., polycytidylic acid) is also included in the cytidylic acid polymer in the present invention.

The cytidylic acid copolymer is a polymer having a structural unit derived from CMP and a structural unit derived from a nucleotide other than CMP.

The nucleotide other than CMP cannot be limited, and may be a ribonucleotide or a deoxyribonucleotide. Examples of nucleotides other than CMP include IMP, dIMP, dCMP, GMP, dGMP, AMP, dAMP, TMP, dTMP, UMP, dUMP, and a combination of two or more thereof. Of these, at least one member selected from the group consisting of IMP, dIMP, dCMP, GMP, dGMP, AMP, and dAMP is preferable, at least one member selected from the group consisting of IMP, dIMP, dCMP, GMP, and dGMP is more preferably, and at least one member selected from the group consisting of IMP and dIMP is more preferable.

The "derivative" and "salt" are as described in section (1) Inosinic Acid Polymer.

In the cytidylic acid polymer, the structural unit derived from CMP is present in an amount of, for example, over 50 mol%, preferably 60 mol% or more, more preferably 65 mol% or more, more preferably 70 mol% or more, more preferably 75 mol% or more, more preferably 80 mol% or more, more preferably 85 mol% or more, more preferably 90 mol% or more, or more preferably 95 mol% or more in the total structural units. The structural unit derived from CMP may also be present in an amount of 100 mol% (i.e., a homopolymer), or for example, 99.9 mol% or less, or 99 mol% or less in the total structural units.

In the cytidylic acid polymer, the number of repetitions of the structural unit derived from CMP (this can also be expressed as, for example, a base length) may be, for example, 30 or more, preferably 40 or more, more preferably 50 or more, still more preferably 60 or more, yet more preferably 70 or more, for example, 100 or more, or 150 or more. The number of repetitions of the structural unit derived from CMP may also be, for example, 10000 or less, 8000 or less, 5000 or less, 3000 or less, 1000 or less, for example, less than 100, 95 or less, or 90 or less. The repetition may be continuous or intermittent, and preferably continuous.

### (3) Guanylic Acid Polymer

The guanylic acid polymer as used here includes the meanings of a guanylic acid homopolymer (polyguanylic acid), a guanylic acid copolymer, derivatives thereof, and salts thereof. A double-stranded nucleic acid polymer of which at least one strand is a guanylic acid polymer (e.g., polyguanylic acid) is also included in the guanylic acid polymer in the present invention.

The guanylic acid copolymer is a polymer having a structural unit derived from GMP and a structural unit derived from a nucleotide other than GMP. The nucleotide other than GMP cannot be limited, and may be a ribonucleotide or a deoxyribonucleotide. Examples of nucleotides other than GMP include IMP, dIMP, CMP, dCMP, dGMP, AMP, dAMP, TMP, dTMP, UMP, dUMP, and a combination of two or more thereof. Of these, at least one member selected from the group consisting of IMP, dIMP, CMP, dCMP, dGMP, AMP, and dAMP is preferable, at least one member selected from the group consisting of IMP, dIMP, CMP, dCMP, and dGMP is more preferable, and at least one member selected from the group consisting of IMP and dIMP is more preferable.

The "derivative" and "salt" are as described in section (1) Inosinic Acid Polymer.

In the guanylic acid polymer, the structural unit derived from GMP is present in an amount of, for example, over 50 mol%, preferably 60 mol% or more, more preferably 65 mol% or more, more preferably 70 mol% or more, more preferably 75 mol% or more, more preferably 80 mol% or more, more preferably 85 mol% or more, more preferably 90 mol% or more, or more preferably 95 mol% or more in the total structural units. The structural unit derived from GMP may also be present in an amount of 100 mol% (i.e., a homopolymer), or for example, 99.9 mol% or less, or 99 mol% or less in the total structural units.

In the guanylic acid polymer, the number of repetitions of the structural unit derived from GMP (this can also be expressed as, for example, a base length) may be, for example, 30 or more, preferably 40 or more, more preferably 50 or more, still more preferably 60 or more, yet more preferably 70 or more, for example, 100 or more, or 150 or more. The number of repetitions of the structural unit derived from GMP may also be, for example, 10000 or less, 8000 or less, 5000 or less, 3000 or less, 1000 or less, for example, less than 100, 95 or less, or 90 or less. The repetition may be continuous or intermittent, and preferably continuous.

### (4) Adenylic Acid Polymer

The adenylic acid polymer as used here includes the meanings of an adenylic acid homopolymer (polyadenylic acid), an adenylic acid copolymer, derivatives thereof, and salts thereof. A double-stranded nucleic acid polymer of which at least one strand is an adenylic acid polymer (e.g., polyadenylic acid) is also included in the adenylic acid polymer in the present invention.

The adenylic acid copolymer is a polymer having a structural unit derived from AMP and a structural unit derived from a nucleotide other than AMP.

The nucleotide other than AMP cannot be limited, and may be a ribonucleotide or a deoxyribonucleotide. Examples of nucleotides other than AMP include IMP, dIMP, CMP, dCMP, GMP, dGMP, dAMP, TMP, dTMP, UMP, dUMP, and a combination of two or more thereof. Of these, at least one member selected from the group consisting of IMP, dIMP, CMP, dCMP, GMP, dGMP, and dAMP is preferable, at least one member selected from the group consisting of IMP, dIMP, CMP, dCMP, GMP, and dGMP is more preferable, and at least one member selected from the group consisting of IMP and dIMP is more preferable.

The "derivative" and "salt" are as described in section (1) Inosinic Acid Polymer.

In the adenylic acid polymer, the structural unit derived from AMP is present in an amount of, for example, over 50 mol%, preferably 60 mol% or more, more preferably 65 mol% or more, more preferably 70 mol% or more, more preferably 75 mol% or more, more preferably 80 mol% or more, more preferably 85 mol% or more, more preferably 90 mol% or more, or more preferably 95 mol% or more in the total structural units. The structural unit derived from AMP may also be present in an amount of 100 mol% (i.e., a homopolymer), or for example, 99.9 mol% or less, or 99 mol% or less in the total structural units.

In the adenylic acid polymer, the number of repetitions of the structural unit derived from AMP (this can also be expressed as, for example, a base length) may be, for example, 30 or more, preferably 40 or more, more preferably 50 or more, still more preferably 60 or more, yet more preferably 70 or more, for example, 100 or more, or 150 or more. The number of repetitions of the structural unit derived from AMP can also be, for example, 10000 or less, 8000 or less, 5000 or less, 3000 or less, or 1000 or less. The repetition may be continuous or intermittent, and preferably continuous.

### (5) Thymidylic Acid Polymer

The thymidylic acid polymer as used here includes the meanings of a thymidylic acid homopolymer (polythymidylic acid), a thymidylic acid copolymer, derivatives thereof, and salts thereof. A double-stranded nucleic acid polymer of which at least one strand is a thymidylic acid polymer (e.g., polythymidylic acid) is also included in the thymidylic acid polymer in the present invention.

The thymidylic acid copolymer is a polymer having a structural unit derived from TMP and a structural unit derived from a nucleotide other than TMP.

The nucleotide other than TMP cannot be limited, and may be a ribonucleotide or a deoxyribonucleotide. Examples of nucleotides other than TMP include IMP, dIMP, CMP, dCMP, GMP, dGMP, AMP, dAMP, dTMP, UMP, dUMP, and a combination of two or more thereof. Of these, at least one member selected from the group consisting of IMP, dIMP, CMP, dCMP, GMP, dGMP, AMP, and dAMP is preferable, at least one member selected from the group consisting of IMP, dIMP, CMP, dCMP, GMP, and dGMP is more preferable, and at least one member selected from the group consisting of IMP and dIMP is more preferable.

The "derivative" and "salt" are as described in section (1) Inosinic Acid Polymer.

In the thymidylic acid polymer, the structural unit derived from TMP is present in an amount of, for example, over 50 mol%, preferably 60 mol% or more, more preferably 65 mol% or more, more preferably 70 mol% or more, more preferably 75 mol% or more, more preferably 80 mol% or more, more preferably 85 mol% or more, more preferably 90 mol% or more, or more preferably 95 mol% or more in the total structural units. The structural unit derived from TMP may also be present in an amount of 100 mol% (i.e., a homopolymer), or for example, 99.9 mol% or less, or 99 mol% or less in the total structural units.

In the thymidylic acid polymer, the number of repetitions of the structural unit derived from TMP (this can also be expressed as, for example, a base length) may be, for example, 30 or more, preferably 40 or more, more preferably 50 or more, still more preferably 60 or more, yet more preferably 70 or more, for example, 100 or more, or 150 or more. The number of repetitions of the structural unit derived from TMP may also be, for example, 10000 or less, 8000 or less, 5000 or less, 3000 or less, 1000 or less, for example, less than 100, 95 or less, or 90 or less. The repetition may be continuous or intermittent, and preferably continuous.

### (6) Uridylic Acid Polymer

The uridylic acid polymer as used here includes the meanings of an uridylic acid homopolymer (polyuridylic acid), an uridylic acid copolymer, derivatives thereof, and salts thereof. A double-stranded nucleic acid polymer of which at least one strand is an uridylic acid polymer (e.g., polyuridylic acid) is also included in the uridylic acid polymer in the present invention.

The uridylic acid copolymer is a polymer having a structural unit derived from UMP and a structural unit derived from a nucleotide other than UMP.

The nucleotide other than UMP cannot be limited, and may be a ribonucleotide or a deoxyribonucleotide. Examples of nucleotides other than UMP include IMP, dIMP, CMP, dCMP, GMP, dGMP, AMP, dAMP, TMP, dTMP, dUMP, and a combination of two or more thereof. Of these, at least one member selected from the group consisting of IMP, dIMP, CMP, dCMP, GMP, dGMP, AMP, and dAMP is preferable, at least one member selected from the group consisting of IMP, dIMP, CMP, dCMP, GMP, and dGMP is more preferable, and at least one member selected from the group consisting of IMP and dIMP is more preferable.

The "derivative" and "salt" are as described in section (1) Inosinic Acid Polymer.

In the uridylic acid polymer, the structural unit derived from UMP is present in an amount of, for example, over 50 mol%, preferably 60 mol% or more, more preferably 65 mol% or more, more preferably 70 mol% or more, more preferably 75 mol% or more, more preferably 80 mol% or more, more preferably 85 mol% or more, more preferably 90 mol% or more, or more preferably 95 mol% or more in the total structural units. The structural unit derived from UMP may also be present in an amount of 100 mol% (i.e., a homopolymer), or for example, 99.9 mol% or less, or 99 mol% or less in the total structural units.

In the uridylic acid polymer, the number of repetitions of the structural unit derived from UMP (this can also be expressed as, for example, a base length) may be, for example, 30 or more, preferably 40 or more, more preferably 50 or more, still more preferably 60 or more, yet more preferably 70 or more, for example, 100 or more, or 150 or more. The number of repetitions of the structural unit derived from UMP can also be, for example, 10000 or less, 8000 or less, 5000 or less, 3000 or less, or 1000 or less. The repetition may be continuous or intermittent, and preferably continuous.

### (7) Deoxyinosinic Acid Polymer

The deoxyinosinic acid polymer as used here includes the meanings of a deoxyinosinic acid homopolymer (polydeoxyinosinic acid), a deoxyinosinic acid copolymer, derivatives thereof, and salts thereof. A double-stranded nucleic acid polymer of which at least one strand is a deoxyinosinic acid polymer (e.g., polydeoxyinosinic acid) is also included in the deoxyinosinic acid polymer in the present invention.

The deoxyinosinic acid copolymer is a polymer having a structural unit derived from dIMP and a structural unit derived from a nucleotide other than dIMP.

The nucleotide other than dIMP cannot be limited, and may be a ribonucleotide or a deoxyribonucleotide. Examples of nucleotides other than dIMP include IMP, CMP, dCMP, GMP, dGMP, AMP, dAMP, TMP, dTMP, UMP, dUMP, and a combination of two or more thereof. Of these, at least one member selected from the group consisting of IMP, CMP, dCMP, GMP, dGMP, AMP, and dAMP is preferable, at least one member selected from the group consisting of IMP, CMP, dCMP, GMP, and dGMP is more preferable, and IMP is more preferable.

The "derivative" and "salt" are as described in section (1) Inosinic Acid Polymer.

In the deoxyinosinic acid polymer, the structural unit derived from dIMP is present in an amount of, for example, over 50 mol%, preferably 60 mol% or more, more preferably 65 mol% or more, more preferably 70 mol% or more, more preferably 75 mol% or more, more preferably 80 mol% or more, more preferably 85 mol% or more, more preferably 90 mol% or more, or more preferably 95 mol% or more in the total structural units. The structural unit derived from dIMP may also be present in an amount of 100 mol% (i.e., a homopolymer), or for example, 99.9 mol% or less, or 99 mol% or less in the total structural units.

In the deoxyinosinic acid polymer, the number of repetitions of the structural unit derived from dIMP (this can also be expressed as, for example, a base length) may be, for example, 30 or more, preferably 40 or more, more preferably 50 or more, still more preferably 60 or more, yet more preferably 70 or more, for example, 100 or more, or 150 or more. The number of repetitions of the structural unit derived from dIMP may also be, for example, 10000 or less, 8000 or less, 5000 or less, 3000 or less, or 1000 or less. The repetition may be continuous or intermittent, and preferably continuous.

### (8) Deoxycytidylic Acid Polymer

The deoxycytidylic acid polymer as used here includes the meanings of a deoxycytidylic acid homopolymer (polydeoxycytidylic acid), a deoxycytidylic acid copolymer, derivatives thereof, and salts thereof. A double-stranded nucleic acid polymer of which at least one strand is a deoxycytidylic acid polymer (e.g., polydeoxycytidylic acid) is also included in the deoxycytidylic acid polymer in the present invention.

The deoxycytidylic acid copolymer is a polymer having a structural unit derived from dCMP and a structural unit derived from a nucleotide other than dCMP.

The nucleotide other than dCMP cannot be limited, and may be a ribonucleotide or a deoxyribonucleotide. Examples of nucleotides other than dCMP include IMP, dIMP, CMP, GMP, dGMP, AMP, dAMP, TMP, dTMP, UMP, dUMP, and a combination of two or more thereof. Of these, at least one member selected from the group consisting of IMP, dIMP, CMP, GMP, dGMP, AMP, and dAMP is preferable, at least one member selected from the group consisting of IMP, dIMP, CMP, GMP, and dGMP is more preferable, and at least one member selected from the group consisting of IMP and dIMP is more preferable.

The "derivative" and "salt" are as described in section (1) Inosinic Acid Polymer.

In the deoxycytidylic acid polymer, the structural unit derived from dCMP is present in an amount of, for example, over 50 mol%, preferably 60 mol% or more, more preferably 65 mol% or more, more preferably 70 mol% or more, more preferably 75 mol% or more, more preferably 80 mol% or more, more preferably 85 mol% or more, more preferably 90 mol% or more, or more preferably 95 mol% or more in the total structural units. The structural unit derived from dCMP may also be present in an amount of 100 mol% (i.e., a homopolymer), or for example, 99.9 mol% or less, or 99 mol% or less in the total structural units.

In the deoxycytidylic acid polymer, the number of repetitions of the structural unit derived from dCMP (this can also be expressed as, for example, a base length) may be, for example, 30 or more, preferably 40 or more, more preferably 50 or more, still more preferably 60 or more, yet more preferably 70 or more, for example, 100 or more, or 150 or more. The number of repetitions of the structural unit derived from dCMP may also be, for example, 10000 or less, 8000 or less, 5000 or less, 3000 or less, 1000 or less, for example, less than 100, 95 or less, or 90 or less. The repetition may be continuous or intermittent, and preferably continuous.

### (9) Deoxyguanylic Acid Polymer

The deoxyguanylic acid polymer as used here includes the meanings of a deoxyguanylic acid homopolymer (polydeoxyguanylic acid), a deoxyguanylic acid copolymer, derivatives thereof, and salts thereof. A double-stranded nucleic acid polymer of which at least one strand is a deoxyguanylic acid polymer (e.g., polydeoxyguanylic acid) is also included in the deoxyguanylic acid polymer in the present invention.

The deoxyguanylic acid copolymer is a polymer having a structural unit derived from dGMP and a structural unit derived from a nucleotide other than dGMP.

The nucleotide other than dGMP cannot be limited, and may be a ribonucleotide or a deoxyribonucleotide. Examples of nucleotides other than dGMP include IMP, dIMP, CMP, dCMP, GMP, AMP, dAMP, TMP, dTMP, UMP, dUMP, and a combination of two or more thereof. Of these, at least one member selected from the group consisting of IMP, dIMP, CMP, dCMP, GMP, AMP, and dAMP is preferable, at least one member selected from the group consisting of IMP, dIMP, CMP, dCMP, and GMP is more preferable, and at least one member selected from the group consisting of IMP and dIMP is more preferable.

The "derivative" and "salt" are as described in section (1) Inosinic Acid Polymer.

In the deoxyguanylic acid polymer, the structural unit derived from dGMP is present in an amount of, for example, over 50 mol%, preferably 60 mol% or more, more preferably 65 mol% or more, more preferably 70 mol% or more, more preferably 75 mol% or more, more preferably 80 mol% or more, more preferably 85 mol% or more, more preferably 90 mol% or more, or more preferably 95 mol% or more in the total structural units. The structural unit derived from dGMP may also be present in an amount of 100 mol% (i.e., a homopolymer), or for example, 99.9 mol% or less, or 99 mol% or less in the total structural units.

In the deoxyguanylic acid polymer, the number of repetitions of the structural unit derived from dGMP (this can also be expressed as, for example, a base length) may be, for example, 30 or more, preferably 40 or more, more preferably 50 or more, still more preferably 60 or more, yet more preferably 70 or more, for example, 100 or more, or 150 or more. The number of repetitions of the structural unit derived from dGMP may also be, for example, 10000 or less, 8000 or less, preferably 6500 or less, more preferably 6000 or less, 5000 or less, 3000 or less, 1000 or less, for example, less than 100, 95 or less, or 90 or less. The repetition may be continuous or intermittent, and preferably continuous.

### (10) Deoxyadenylic Acid Polymer

The deoxyadenylic acid polymer as used here includes the meanings of a deoxyadenylic acid homopolymer (polydeoxyadenylic acid), a deoxyadenylic acid copolymer, derivatives thereof, and salts thereof. A double-stranded nucleic acid polymer of which at least one strand is a deoxyadenylic acid polymer (e.g., polydeoxyadenylic acid) is also included in the deoxyadenylic acid polymer in the present invention.

The deoxyadenylic acid copolymer is a polymer having a structural unit derived from dAMP and a structural unit derived from a nucleotide other than dAMP.

The nucleotide other than dAMP cannot be limited, and may be a ribonucleotide or a deoxyribonucleotide. Examples of nucleotides other than dAMP include IMP, dIMP, CMP, dCMP, GMP, dGMP, AMP, TMP, dTMP, UMP, dUMP, and a combination of two or more thereof. Of these, at least one member selected from the group consisting of IMP, dIMP, CMP, dCMP, GMP, dGMP, and AMP is preferable, at least one member selected from the group consisting of IMP, dIMP, CMP, dCMP, GMP, and dGMP is more preferable, and at least one member selected from the group consisting of IMP and dIMP is more preferable.

The "derivative" and "salt" are as described in section (1) Inosinic Acid Polymer.

In the deoxyadenylic acid polymer, the structural unit derived from dAMP is present in an amount of, for example, over 50 mol%, preferably 60 mol% or more, more preferably 65 mol% or more, more preferably 70 mol% or more, more preferably 75 mol% or more, more preferably 80 mol% or more, more preferably 85 mol% or more, more preferably 90 mol% or more, or more preferably 95 mol% or more in the total structural units. The structural unit derived from dAMP may also be present in an amount of 100 mol% (i.e., a homopolymer), or for example, 99.9 mol% or less, or 99 mol% or less in the total structural units.

In the deoxyadenylic acid polymer, the number of repetitions of the structural unit derived from dAMP (this can also be expressed as, for example, a base length) may be, for example, 30 or more, preferably 40 or more, more preferably 50 or more, still more preferably 60 or more, yet more preferably 70 or more, for example, 100 or more, or 150 or more. The number of repetitions of the structural unit derived from dAMP may also be, for example, 10000 or less, 8000 or less, 5000 or less, 3000 or less, or 1000 or less. The repetition may be continuous or intermittent, and preferably continuous.

### (11) Deoxythymidylic Acid Polymer

The deoxythymidylic acid polymer as used here includes the meanings of a deoxythymidylic acid homopolymer (polydeoxythymidylic acid), a deoxythymidylic acid copolymer, derivatives thereof, and salts thereof. A double-stranded nucleic acid polymer of which at least one strand is a deoxythymidylic acid polymer (e.g., polydeoxythymidylic acid) is also included in the deoxythymidylic acid polymer in the present invention.

The deoxythymidylic acid copolymer is a polymer having a structural unit derived from dTMP and a structural unit derived from a nucleotide other than dTMP.

The nucleotide other than dTMP cannot be limited, and may be a ribonucleotide or a deoxyribonucleotide. Examples of nucleotides other than dTMP include IMP, dIMP, CMP, dCMP, GMP, dGMP, AMP, dAMP, TMP, UMP, dUMP, and a combination of two or more thereof. Of these, at least one member selected from the group consisting of IMP, dIMP, CMP, dCMP, GMP, dGMP, AMP, and dAMP is preferable, at least one member selected from the group consisting of IMP, dIMP, CMP, dCMP, GMP, and dGMP is more preferable, and at least one member selected from the group consisting of IMP and dIMP is more preferable.

The "derivative" and "salt" are as described in section (1) Inosinic Acid Polymer.

In the deoxythymidylic acid polymer, the structural unit derived from dTMP is present in an amount of, for example, over 50 mol%, preferably 60 mol% or more, more preferably 65 mol% or more, more preferably 70 mol% or more, more preferably 75 mol% or more, more preferably 80 mol% or more, more preferably 85 mol% or more, more preferably 90 mol% or more, or more preferably 95 mol% or more in the total structural units. The structural unit derived from dTMP may also be present in an amount of 100 mol% (i.e., a homopolymer), or for example, 99.9 mol% or less, or 99 mol% or less in the total structural units.

In the deoxythymidylic acid polymer, the number of repetitions of the structural unit derived from dTMP (this can also be expressed as, for example, a base length) may be, for example, 30 or more, preferably 40 or more, more preferably 50 or more, preferably 60 or more, more preferably 70 or more, for example, 100 or more, or 150 or more. The number of repetitions of the structural unit derived from dTMP may also be, for example, 10000 or less, 8000 or less, 5000 or less, 3000 or less, or 1000 or less. The repetition may be continuous or intermittent, and preferably continuous.

### (12) Deoxyuridylic Acid Polymer

The deoxyuridylic acid polymer as used here includes the meanings of a deoxyuridylic acid homopolymer (polydeoxyuridylic acid), a deoxyuridylic acid copolymer, derivatives thereof, and salts thereof. A double-stranded nucleic acid polymer of which at least one strand is a deoxyuridylic acid polymer (e.g., polydeoxyuridylic acid) is also included in the deoxyuridylic acid polymer in the present invention.

The deoxyuridylic acid copolymer is a polymer having a structural unit derived from dUMP and a structural unit derived from a nucleotide other than dUMP.

The nucleotide other than dUMP cannot be limited, and may a ribonucleotide or a deoxyribonucleotide. Examples of nucleotides other than dUMP include IMP, dIMP, CMP, dCMP, GMP, dGMP, AMP, dAMP, TMP, dTMP, UMP, and a combination of two or more thereof. Of these, at least one member selected from the group consisting of IMP, dIMP, CMP, dCMP, GMP, dGMP, AMP, and dAMP is preferable, at least one member selected from the group consisting of IMP, dIMP, CMP, dCMP, GMP, and dGMP is more preferable, and at least one member selected from the group consisting of IMP and dIMP is more preferable.

The "derivative" and "salt" are as described in section (1) Inosinic Acid Polymer.

In the deoxyuridylic acid polymer, the structural unit derived from dUMP is present in an amount of, for example, over 50 mol%, preferably 60 mol% or more, more preferably 65 mol% or more, more preferably 70 mol% or more, more preferably 75 mol% or more, more preferably 80 mol% or more, more preferably 85 mol% or more, more preferably 90 mol% or more, or more preferably 95 mol% or more in the total structural units. The structural unit derived from dUMP may also be present in an amount of 100 mol% (i.e., a homopolymer), or for example, 99.9 mol% or less, or 99 mol% or less in the total structural units.

In the deoxyuridylic acid polymer, the number of repetitions of the structural unit derived from dUMP (this can also be expressed as, for example, a base length) may be, for example, 30 or more, preferably 40 or more, more preferably 50 or more, preferably 60 or more, more preferably 70 or more, for example, 100 or more, or 150 or more. The number of repetitions of the structural unit derived from dUMP may also be, for example, 10000 or less, 8000 or less, 5000 or less, 3000 or less, or 1000 or less. The repetition may be continuous or intermittent, and preferably continuous.

In a preferable embodiment, the nucleic acid polymer contains a structural unit derived from a nucleotide selected from the group consisting of IMP, dIMP, CMP, dCMP, GMP, dGMP, derivatives thereof, and salts thereof in an amount of, for example, 60 mol% or more, preferably 65 mol% or more, more preferably 70 mol% or more, more preferably 75 mol% or more, more preferably 80 mol% or more, more preferably 85 mol% or more, more preferably 90 mol% or more, or more preferably 95 mol% or more of the total structural units. The upper limit can be any value; for example, the nucleic acid polymer may contain these structural units in an amount of 100 mol%, 99.9 mol% or less, or 99 mol% or less of the total structural units.

In a more preferable embodiment, the nucleic acid polymer contains a structural unit derived from a nucleotide selected from the group consisting of IMP, dIMP, derivatives thereof, and salts thereof in an amount of, for example, 60 mol% or more, preferably 65 mol% or more, more preferably 70 mol% or more, more preferably 75 mol% or more, more preferably 80 mol% or more, more preferably 85 mol% or more, more preferably 90 mol% or more, or more preferably 95 mol% or more of the total structural units. The upper limit can be any value; for example, the nucleic acid polymer may contain these structural units in an amount of 100 mol%, 99.9 mol% or less, or 99 mol% or less of the total structural units.

In a preferable embodiment, the nucleic acid polymer is at least one member selected from the group consisting of polyinosinic acid, polycytidylic acid, polyguanylic acid, polyadenylic acid, polythymidylic acid, polyuridylic acid, polydeoxyinosinic acid, polydeoxycytidylic acid, polydeoxyguanylic acid, polydeoxyadenylic acid, polydeoxythymidylic acid, polydeoxyuridylic acid, and salts thereof.

In a more preferable embodiment, the nucleic acid polymer is at least one member selected from the group consisting of polyinosinic acid, polycytidylic acid, polyguanylic acid, polyadenylic acid, polydeoxyinosinic acid, polydeoxycytidylic acid, polydeoxyguanylic acid, polydeoxyadenylic acid, and salts thereof.

In a more preferable embodiment, the nucleic acid polymer is at least one member selected from the group consisting of polyinosinic acid, polycytidylic acid, polyguanylic acid, polydeoxyinosinic acid, polydeoxycytidylic acid, polydeoxyguanylic acid, and salts thereof.

In a more preferable embodiment, the nucleic acid polymer is at least one member selected from the group consisting of polyinosinic acid, polydeoxyinosinic acid, and salts thereof.

The above nucleic acid polymer preferably includes the above-described nucleic acid polymer having a total length of 25 to 15000 bases, preferably 30 to 10000 bases; more preferably the nucleic acid polymer having a total length of 40 to 9000 bases; more preferably the nucleic acid polymer having a total length of 50 to 8000 bases; or for example, the above-described nucleic acid polymer having a length of 100 to 5000 bases. For example, the nucleic acid polymer having a total length of 25 to 15000 bases, 30 to 10000 bases, 40 to 9000 bases, 50 to 8000 bases, or 100 to 5000 bases is present in an amount of, for example, 70 mol% or more, preferably 80 mol% or more, more preferably 85 mol% or more, more preferably 90 mol% or more, or more preferably 95 mol% or more based on the entire nucleic acid polymer.

Specifically, the nucleic acid polymer may be a mixture of nucleic acid polymers of various base lengths, such as a mixture of nucleic acid polymers having a length substantially distributed within the range of 25 to 15000 bases, preferably a mixture of nucleic acid polymers having a length substantially distributed within the range of 30 to 10000 bases, more preferably a mixture of nucleic acid polymers having a length substantially distributed within the range of 40 to 9000 bases, or still more preferably a mixture of nucleic acid polymers having a length substantially distributed within the range of 50 to 8000 bases. For example, a mixture of nucleic acid polymers having a length substantially distributed within the range of 100 to 5000 bases is also usable. The phrase "distributed substantially within the range of A to B bases" means that nucleic acid polymers having a base length of A to B account for, for example, 70 mol% or more of the entire nucleic acid polymer.

The nucleic acid polymer may be, for example, a mixture of nucleic acid polymers having such a distribution that a peak is present around a length of 30 to 3000 bases (or mean or mode), a mixture of nucleic acid polymers having such a distribution that a peak is present around a length of 50 to 1500 bases (or mean or mode), and preferably a mixture of nucleic acid polymers having such a distribution that a peak is present around a length of 60 to 1000 bases (or mean or mode). A mixture of nucleic acid polymers having such a distribution that a peak is present around a length of 70 to 800 bases (or mean or mode) is also usable. The term "around" means that the lower limit and/or the upper limit may be increased or decreased by 10%.
The nucleic acid polymer may have a total length of, for example, 30 bases or more, preferably 40 bases or more, more preferably 50 bases or more, still more preferably 60 bases or more, and yet more preferably 70 bases or more; for example, the nucleic acid polymer may have a total length of 100 bases or more, and 150 bases or more. As described above, the nucleic acid polymer preferably has a total length longer than the base length designed for a probe or primer. The nucleic acid polymer may have a total length of, for example, 10000 bases or less, 8000 bases or less, or, for example, 5000 bases or less.

The nucleic acid polymer can be synthesized by any method usable in synthesizing an oligonucleotide, such as a phosphotriester method, an H-phosphonate method, or a thiophosphonate method. The nucleic acid polymer for use may be a natural product, or preferably a commercially available product. Examples of commercially available nucleic acid polymers for use include a polyinosinic acid potassium salt produced by Sigma-Aldrich, a polyadenylic acid potassium salt produced by Sigma-Aldrich, and a polyinosinic acid potassium salt produced by Santa Cruz Biotechnology.

### 2. Method for Synthesizing DNA by Reverse Transcription of Template RNA

The method for synthesizing DNA by reverse transcription of template RNA can be any method, and may be selected from a variety of known methods. The method typically includes the step of incubating a composition for reverse transcription (or solution for reverse transcription reaction) containing a protein that has reverse transcription activity, such as a reverse transcriptase.

### 2-1. Composition for Reverse Transcription

The composition for reverse transcription contains, for example, the nucleic acid polymer described above, template RNA, a primer, a deoxyribonucleotide, and a reverse transcriptase. The composition for reverse transcription may optionally contain other components, such as a DNA polymerase.

From the standpoint of suppressing the reverse transcription of rRNA and suppressing the synthesis of DNA derived from rRNA, the concentration of the nucleic acid polymer in the composition for reverse transcription may be, for example, 0.1 ng/µl or more, preferably 0.2 ng/µl or more, more preferably 1 ng/µl or more, still more preferably 1.5 ng/µl or more, for example, 2 ng/µl or more, 3 ng/µl or more, 4 ng/µl or more, or 5 ng/µl or more. The concentration of the nucleic acid polymer may be, for example, 50 ng/µl or less, preferably 25 ng/µl or less, more preferably 20 ng/µl or less, for example, 10 ng/µl or less, 5 ng/µl or less, 3 ng/µl or less, 2.5 ng/µl or less, 2 ng/µl or less, 1 ng/µl or less, or less than 1 ng/µl. Even by using a nucleic acid polymer in such a low concentration, the present invention can suppress the reverse transcription of rRNA and the synthesis of DNA derived from rRNA.

The content of the nucleic acid polymer is, for example, 100 to 50000 parts by mass, preferably 1000 to 10000 parts by mass, or more preferably 1000 to 5000 parts by mass, per 100 parts by mass of the total amount of RNA (including rRNA) contained in the composition for reverse transcription.

### (1) Template RNA

The template RNA can be any RNA such as RNA extracted from tissue or cells (e.g., extracted RNA treated with an extraction technique known in the art, such as phenol chloroform extraction), or RNA extracted from tissue or cells and then further purified (e.g., purified RNA treated with a purification technique known in the art, such as ethanol precipitation or column purification). In particular, the template RNA for use is preferably RNA extracted from cells. Typically, RNA extracted from cells inevitably contain rRNA in addition to RNA for use as template RNA such as mRNA. However, because the reverse transcription of rRNA is suppressed in the presence of the nucleic acid polymer, template RNA can be specifically reverse-transcribed even by using such RNA extracted from cells as template RNA. More specifically, the step of removing rRNA from RNA extracted from cells can be eliminated.

The cells from which RNA is extracted are not particularly limited in type, and can be of any type. The number of cells can be suitably adjusted with a cell sorter. For example, RNA extracted from a small number of cells (e.g. 1 to 100 cells, preferably 1 to 10 cells, more preferably 1 or 2 cells, and more preferably 1 cell) can be used.

The method for extracting RNA from cells typically includes the step of lysing cells by using a composition for cell lysis that contains a cell lysis reagent.
Examples of cell lysis reagents include surfactants and chaotropic agents. Examples of surfactants include anionic surfactants (e.g., sodium dodecyl sulfate, sodium cholate, and sodium deoxycholate), cationic surfactants (e.g., cetyltrimethylammonium bromide), nonionic surfactants (e.g., octylphenol ethoxylate, polyoxyethylene lauryl ether, polyoxyethylene stearyl ether, and polyoxyethylene sorbitan monolaurate), and zwitterionic surfactants (e.g., 3-[(3-cholamidopropyl)dimethylammonio]-1-propanesulfonic acid). Examples of chaotropic agents include urea and lithium salts such as lithium perchlorate. The cell lysis reagent is typically dissolved in water (preferably nuclease-free water) and used in the form of an aqueous solution.

The composition for cell lysis may contain a protease (e.g., protease K), an RNase inhibitor, or a combination of two or more of these components.

The composition for cell lysis may or may not contain the nucleic acid polymer. When RNA extracted from cells by using a composition for cell lysis that contains the above nucleic acid polymer is used as template RNA, the composition for reverse transcription does not have to contain the nucleic acid polymer. The details of the composition for cell lysis are disclosed, for example, in US Patent Application Publication No. 2011/0111463, the entire disclosure of which is incorporated herein by reference.

The concentration of RNA (including rRNA) in the composition for reverse transcription is, for example, 1 to 10000 pg/µl, preferably 10 to 1000 pg/µl, or more preferably 1 to 100 pg/µl.

### (2) Primer

The primer includes a primer specific to template RNA, an oligo-dT primer, a random primer, and a combination of two or more thereof. Of these, an oligo-dT primer and a random primer tend to more easily anneal to any RNA than a primer specific to a particular sequence, and are likely to evoke the synthesis of DNA from rRNA. Even when such an oligo-dT primer or random primer is used, the present invention can effectively suppress the synthesis of DNA from rRNA. From this viewpoint, the present invention is beneficial in performing reverse transcription reaction by using an oligo-dT primer and/or a random primer, and is particularly beneficial in performing reverse transcription reaction by using a combination of an oligo-dT primer and a random primer. The molar ratio of a random primer to an oligo-dT primer (an oligo-dT primer: a random primer) is, for example, 1:5 to 1:15, and preferably 1:8 to 1:12.

Examples of random primers include completely random primers and NSR (not so random) primers.

The completely random primers refer to a mixture of primers of various base sequences, in which each base sequence is completely random. The completely random primers may contain a sequence that is completely identical (or completely complementary) to the sequence of rRNA. Examples of completely random primers include completely random pentamers, completely random hexamers, completely random heptamers, completely random octamers, and combinations thereof. For example, completely random hexamers may be a mixture of all possible base sequences formed by the four nucleotides (A, T, C, and G) (4⁶ types) .

NSR primers refer to primers prepared by removing a primer whose sequence is completely complementary to the sequence of rRNA from completely random primers. Examples of the sequence of rRNA to be removed include the sequence of 18S rRNA, the sequence of 28S rRNA, the sequence of 12S rRNA, the sequence of 16S rRNA, and combinations thereof.

Examples of NSR primers include those prepared by removing a hexamer with a sequence completely complementary to the sequence of rRNA from completely random hexamers. Those prepared by removing a sequence completely complementary to the sequence of rRNA from a primer set such as of completely random pentamers, completely random heptamers, and completely random octamers are also usable as NSR primers.

The use of such NSR primers enables the analysis of RNA such as mRNA to increase sensitivity.

The details of NSR primers are disclosed, for example, in 1) Amour et al., Digital transcriptome profiling using selective hexamer priming for cDNA synthesis, Nature Methods, Vol. 6, No. 9, 2009, pp. 647-649, 2) Ozsolak et al., Digital transcriptome profiling from attomole-level RNA samples, Genome Research, Vol. 20, 2010, pp. 519-525, and 3) US Patent Application Publication No. 2010/0029511, the entire disclosure of which is incorporated herein by reference.

Although NSR primers are designed to be not completely complementary to the base sequence of rRNA, some of the primers can bind to rRNA, thus resulting in a reverse transcription product containing DNA derived from rRNA as well as DNA derived from template RNA. However, the use of the nucleic acid polymer described above can suppress the synthesis of DNA derived from rRNA. Not wishing to be bound by theory, this presumably occurs because the nucleic acid polymer competitively inhibits the binding of primers such as NSR primers to rRNA.

From the standpoint of annealing, the length of the primers is, for example, 5 bases or more, and preferably 6 bases or more; from the standpoint of synthesis, the length of the primers is, for example, 30 bases or less, preferably 25 bases or less, or more preferably 20 bases or less.

The primers in the composition for reverse transcription can be of any concentration; the concentration of the primers is, for example, 1 to 10 µM, preferably 2 to 6 µM, or more preferably 3 to 5 µM.

### (3) Deoxyribonucleotide

The deoxyribonucleotide is preferably deoxyribonucleoside triphosphate. Examples of deoxyribonucleoside triphosphate include deoxycytidine triphosphate (dCTP), deoxyguanosine triphosphate (dGTP), deoxyadenosine triphosphate (dATP), deoxythymidine triphosphate (dTTP), deoxyuridine triphosphate (dUTP), derivatives thereof, and combinations of two or more thereof. Of these, for example, a mixture of dCTP, dGTP, dATP, and dTTP, a mixture of dCTP, dGTP, dATP, and dUTP, and a mixture of dCTP, dGTP, dATP, dTTP, and dUTP are preferable.

### (4) Reverse Transcriptase

The reverse transcriptase refers to any protein (enzyme) that has reverse transcription activity (RNA-dependent DNA polymerase activity); the reverse transcriptase can be any reverse transcriptase, and is preferably a polymerase that has reverse transcriptase activity. The reverse transcriptase is preferably one that has low RNase H activity or no RNase H activity. Examples of reverse transcriptases include avian myeloblastosis virus reverse transcriptase (AMV-RT), Moloney murine leukemia virus reverse transcriptase (MMLV-RT), human immunovirus reverse transcriptase (HIV-RT), EIRV-RT, RAV2-RT, C. hydrogenogormans DNA polymerase, rTthDNA polymerase, SuperScript I, SuperScript II, variants thereof, and derivatives thereof. Of these, MMLV-RT is preferable.

### (5) DNA Polymerase

The composition for reverse transcription may contain the following DNA polymerases or contain no such DNA polymerases: Taq, Tbr, Tfl, Tru, Tth, Tli, Tac, Tne, Tma, Tih, Tfi, Pfu, Pwo, Kod, Bst, Sac, Sso, Poc, Pab, Mth, Pho, ES4, VENT (trademark), DEEPVENT (trademark), and variants thereof.

### (6) RNase Inhibitor

The composition for reverse transcription may contain an RNase inhibitor. The RNase inhibitor can be any RNase inhibitor. Examples of RNase inhibitors include human placenta-derived proteins, rat lung-derived proteins, and swine liver-derived proteins.

### (7) Additives

The composition for reverse transcription may contain additives. Examples of additives include buffers, salts, and combinations of two or more thereof.

Examples of buffers include Tris, Tricine, Bis-Tricine, Hepes, Mops, Tes, Taps, Pipes, Caps, and mixtures of two or more thereof. The buffer is typically dissolved in water (preferably nuclease-free water) and used in the form of an aqueous solution.

Examples of salts include chlorides (e.g., lithium chloride, sodium chloride, potassium chloride, magnesium chloride, and manganese chloride), acetates (e.g., lithium acetate, sodium acetate, potassium acetate, magnesium acetate, and manganese acetate), and sulfates (e.g., potassium sulfate, magnesium sulfate, and manganese sulfate), and combinations of two or more thereof.

### 2-2. Incubation

The conditions for incubating the composition for reverse transcription are not particularly limited as long as the reverse transcription of template RNA proceeds, and any conditions known in the art can be applied. The temperature of incubation is, for example, 30 to 65°C, and preferably 35 to 60°C. The time period for incubation is, for example, 5 to 120 minutes, and preferably 10 to 60 minutes.

### 2-3. A case in which reverse transcription of template RNA is performed by reverse transcription amplification to amplify cDNA by using RNA as a template (also called "RT-RamDA method")

The RT-RamDA method refers to a nucleic acid amplification method that includes the step of incubating a mixture that contains template RNA, a primer, a degrading enzyme specific to DNA strand in RNA:DNA hybrid strand, an RNase H minus reverse transcriptase, and a substrate. In the RT-RamDA method, due to RNA-dependent DNA polymerase activity of the RNase H minus reverse transcriptase, complementary strand DNA (cDNA) of the template RNA is synthesized, and the cDNA strand of the hybrid strand of RNA and cDNA is randomly cleaved by the degrading enzyme specific to DNA strand in RNA:DNA hybrid strand. With the cleavage site as a starting point, the cDNA strand at the 3' side is peeled off from RNA due to strand displacement activity of the RNase H minus reverse transcriptase, and a new cDNA strand is synthesized at the portion peeled off by the RNase H minus reverse transcriptase. The details of the RT-RamDA method are disclosed, for example, in US Patent Application Publication No. 2017/0275685, the entire disclosure of which is incorporated herein by reference.

When reverse transcription of template RNA is performed by the RT-RamDA method, the composition for reverse transcription contains a degrading enzyme specific to DNA strand in RNA:DNA hybrid strand.

The degrading enzyme specific to DNA strand in RNA:DNA hybrid strand preferably has activity of cleaving the DNA strand in a hybrid strand of RNA and DNA. The enzyme for use may be, for example, a double strand-specific DNA degrading enzyme or a non-specific DNA degrading enzyme.

The double-strand-specific nuclease (or DSN) for use may be a nuclease derived from a prokaryote or a eukaryote, and is preferably a double strand-specific DNA degrading enzyme derived from a crustacean or a modified product thereof. Specific examples include the following:
- Solenocera melantho DNase
- Penaeus japonicus DNase
- Paralithodes camtschaticus DSN
- Pandalus borealis dsDNase
- Chionoecetes opilio DSN
- Other DSN homologs

The double strand-specific DNA degrading enzyme preferably has DNA-degrading activity even at a temperature below 60°C. Of the DNA degrading enzymes above, double strand-specific DNA degrading enzymes derived from a prawn or shrimp or a modified product thereof are preferable.

The double strand-specific DNA degrading enzyme for use can be a commercially available product. Examples of commercially available products of double strand-specific DNA degrading enzymes include dsDNase (ArcticZymes), Hl-dsDNase (ArcticZymes), dsDNase (Thermo Scientific), Shrimp DNase, Recombinant (Affymetrix), Atlantis dsDNase (Zymo Research), and Thermolabile Nuclease (Roche).

The non-specific DNA degrading enzyme is, for example, an enzyme that has activity of cleaving the DNA strand of a hybrid strand of RNA and DNA, and substantially has no activity of cleaving the RNA strand of a hybrid strand of RNA and DNA and a single-stranded RNA, and preferably an enzyme that has activity of cleaving a single-stranded DNA relatively lower than the activity of cleaving the DNA strand of a hybrid strand of RNA and DNA. The non-specific DNA degrading enzyme preferably has DNA-degrading activity even at a temperature below 60°C. The non-specific DNA degrading enzyme for use may be a commercially available product. Examples of non-specific DNA degrading enzymes for use include DNaseI (Thermo Fisher, DNase I). The non-specific DNA degrading enzyme for use may be an enzyme derived from a prokaryote or a eukaryote, and is preferably a non-specific DNA degrading enzyme derived from a mammal or a modified product thereof, more preferably a bovine-derived non-specific DNA degrading enzyme or a modified product thereof.

The "modified product" above refers to an enzyme obtained by modifying a naturally occurring amino acid sequence. Specifically, a modified product refers to an enzyme having an amino acid sequence that is at least 80% (preferably at least 90%, more preferably at least 95%) identical to a naturally occurring amino acid sequence, and an enzyme having an amino acid sequence that contains one or a few (e.g., 1 to 10, preferably 1 to 5, more preferably 1 to 3) deletions, substitutions, and/or additions of amino acids compared with a neutrally occurring amino acid sequence.

When reverse transcription of template RNA is performed by the RT-RamDA method, the composition for reverse transcription may contain a single-stranded-DNA binding protein. The single-stranded-DNA binding protein is typically used together with a degrading enzyme specific to DNA strand in RNA:DNA hybrid strand. Examples of single-stranded-DNA binding proteins include T4 Gene 32 Protein, RecA, SSB (Single-Stranded DNA Binding Protein), and combinations of two or more thereof.

When reverse transcription of template RNA is performed by the RT-RamDA method, the composition for reverse transcription may be incubated under isothermal conditions or under thermal cycling conditions.

### (1) Isothermal Conditions

Incubation under isothermal conditions can be performed, for example, at a predetermined temperature of 25°C or higher and below 50°C, preferably at a predetermined temperature between 30 and 45°C, more preferably at a predetermined temperature between 35 and 40°C, for example, at 37°C for a predetermined period of time (e.g., 5 to 180 minutes, preferably 10 to 150 minutes).

Incubation at a predetermined temperature of 25°C or higher and below 50°C may be performed in two or more steps. For example, incubation can be performed at a predetermined temperature of 25°C or higher and below 30°C for 5 to 15 minutes, and then performed at a predetermined temperature of 30°C or higher and below 35°C for 5 to 15 minutes, followed by incubation at a predetermined temperature of 35°C or higher and below 50°C for a predetermined period of time (e.g., 5 to 60 minutes).

After incubation at a predetermined temperature of 25°C or higher and below 50°C, incubation may be performed, for example, at a predetermined temperature of 50°C or higher and below 100°C. The incubation at a predetermined temperature of 50°C or higher and below 100°C may be performed in two or more steps. For example, incubation may be performed at a predetermined temperature of 50°C or higher and below 80°C for 5 to 15 minutes, and then performed at a predetermined temperature between 80 to 90°C for 5 to 15 minutes.

### (2) Thermal Cycling Conditions

Incubation under thermal cycling conditions can be performed, for example, at predetermined temperature T1 that is 20°C or higher and below 30°C (e.g., 25°C) and at predetermined temperature T2 that is between 30 and 45°C (e.g., 37°C) in combination for a predetermined period of time for T1 (e.g., 1 to 3 minutes; 2 minutes, for example) and for a predetermined period of time for T2 (e.g., 1 to 3 minutes; 2 minutes, for example). This operation can be taken as one cycle and may be performed preferably 10 to 40 cycles, and more preferably 15 to 35 cycles. Before the thermal cycling, incubation may be performed, for example, at a predetermined temperature of 25°C or higher and below 30°C for a predetermined period of time (e.g., 5 to 15 minutes) and performed at a predetermined temperature of 30°C or higher and below 35°C for a predetermined period of time (e.g., 5 to 15 minutes), followed by incubation at a predetermined temperature of 35°C or higher and below 50°C for a predetermined period of time (e.g., 1 to 5 minutes). After the above thermal cycling, incubation may be performed, for example, at a predetermined temperature of 50°C or higher and below 80°C for a predetermined period of time (e.g., 5 to 15 minutes) and performed at a predetermined temperature between 80 and 90°C for a predetermined period of time (e.g., 5 to 15 minutes).

### 2-4. A case in which reverse transcription of template RNA is performed by RT-PCR

When reverse transcription of template RNA is performed by an RT-PCR method, the composition for reverse transcription can be incubated under the conditions described in section 2-2 above. Thereafter, the reverse transcriptase may be optionally inactivated. The method for inactivating reverse transcription cannot be limited, and may be, for example, a method for performing incubation at 90 to 100°C for a predetermined period of time (e.g., 1 to 10 minutes).

When reverse transcription of template RNA is performed by an RT-PCR method, the method for synthesizing DNA by reverse transcription of template RNA includes the step of amplifying DNA contained in the incubated composition for reverse transcription (which is also referred to as a "reverse transcription product"). The step of amplifying DNA typically includes the step of incubating a composition for DNA amplification under thermal cycling conditions.

The composition for DNA amplification may contain a reverse transcription product as it is, or may contain a reverse transcription product diluted with water, preferably nuclease-free water. For example, the reverse transcription product may be diluted so that the mass is 1/20 to 1/30.

The composition for DNA amplification contains, for example, a primer, a deoxyribonucleotide, and a DNA polymerase in addition to the reverse transcription product. When the composition for reverse transcription described in section 2-1 above contains components necessary for DNA amplification such as a DNA polymerase, the composition for reverse transcription can serve as a composition for DNA amplification as it is, and both compositions are interchangeably referred to.

The primer is preferably a primer specific to DNA (including DNA that is reverse-transcribed from RNA) (a forward primer and a reverse primer).

The deoxyribonucleotide and DNA polymerase for use may be those described in sections 2-1 (3) and (5).

The composition for DNA amplification may contain an anti-DNA polymerase antibody, a reaction buffer, a metal ion (e.g., magnesium ion), a fluorescent dye, a fluorescently labeled probe, or a combination of two or more thereof.

Incubation under thermal cycling conditions may be performed, for example, at a predetermined temperature of 80°C or higher and below 100°C for a predetermined period of time (e.g., 10 to 30 seconds) and performed at a predetermined temperature between 50 and 70°C for a predetermined period of time (e.g., 30 seconds to 2 minutes). This operation can be taken as one cycle and may be performed preferably 10 to 50 cycles, and more preferably 15 to 40 cycles.

### 3. A method for suppressing the synthesis of DNA derived from rRNA by using a nucleic acid polymer

The method for suppressing the synthesis of DNA derived from rRNA by using the nucleic acid polymer described above typically includes the step of reverse-transcribing template RNA in the presence of the above nucleic acid polymer. This step enables template RNA to be specifically reverse-transcribed and DNA derived from template RNA to be specifically synthesized, thus suppressing the synthesis of DNA derived from rRNA. rRNA is preferably 18S rRNA, 28S rRNA, 12S rRNA, 16S rRNA, or a combination of two or more thereof. The conditions for reverse transcription are as described in sections 2-1 to 2-4 above.

### A nucleic acid polymer-containing composition for suppressing the reverse transcription of rRNA (or for suppressing the synthesis of DNA derived from rRNA) and use of a nucleic acid polymer for suppressing the reverse transcription of rRNA (or for suppressing the synthesis of DNA derived from rRNA)

The composition for suppressing the reverse transcription of rRNA preferably contains the nucleic acid polymer described above. In order to suppress the reverse transcription of rRNA, it is preferable to use the nucleic acid polymer above. The reverse transcription of rRNA cannot be limited as along as it is reverse transcription of an RNA sample that contains at least rRNA, and may be reverse transcription of an RNA sample that contains template RNA and rRNA. The RNA sample that contains template RNA and rRNA may be RNA extracted from cells or tissue, RNA that has been extracted from cells or tissue and then further purified, or like RNA, with RNA extracted from cells being preferable. This RNA for use may be the RNA described in section 2-1 (1) above.

The conditions for reverse transcription to be applied may be, for example, the same as the conditions described in sections 2-2 to 2-4.

The composition for suppressing the reverse transcription of rRNA may contain components as described in sections 2-1, 2-2, and 2-3 (in particular, components necessary for reverse transcription) in addition to the above nucleic acid polymer.

The composition for suppressing the reverse transcription of rRNA may be in the form of a mixture of those components or in the form of a kit that contains such components separately.

### A reverse transcription reaction composition for preparing a reverse transcription reaction product for use in next-generation sequencing

The reverse transcription reaction composition for preparing a reverse transcription reaction product for use in next-generation sequencing (NGS analysis: next-generation sequencing, also referred to as "next-generation sequencer analysis") preferably contains the nucleic acid polymer described above. The next-generation sequencing typically refers to a sequencing technology capable of simultaneously performing tremendous sequencing reactions of millions to billions. Examples of next-generation sequencing include a method for performing sequence analysis in parallel by using an amplification technique such as emulsion PCR and bridge PCR and a high-sensitivity detection technique such as one-molecule observation. The equipment for performing next-generation sequencing (sequencer) cannot be limited, and examples include MiSeq, HiSeq, NovaSeq (Illumina, Inc.), Genetic Analyzer V2.0, Ion Proton (Thermo Fisher Scientific), MinION, and PromethION (Nanopore). Next-generation sequencing is disclosed in, for example, US Patent Application Publication No. 2014/178438, the entire disclosure of which is incorporated herein by reference.

The present invention can effectively reduce the reverse transcription reaction product from rRNA that could be noise in large-scale gene expression analysis, for example, in next-generation sequencing. In NGS analysis, because the number of reads obtained in one-time analysis is often limited, it is important to reduce unnecessary reads of DNA derived from rRNA. The present invention is advantageous in terms of costs because the present invention can reduce reads of DNA derived from rRNA that is not intended for analysis, is excellent in increasing the number of useful reads (the number of reads excluding the number of reads of rRNA: gene information amount), and would further reduce the number of times for performing NGS analysis. Thus, the reverse transcription reaction product prepared from an RNA sample that could contain rRNA in accordance with the present invention is suitable for use in next-generation sequencing. The RNA sample that could contain rRNA may be a reverse transcription of an RNA sample that contains template RNA and rRNA. The RNA sample that contains template RNA and rRNA may be RNA extracted from cells or tissue, RNA that has been extracted from cells or tissue and then further purified, or like RNA, with RNA extracted from cells being preferable. This RNA for use may be the same as RNA described in section 2-1 (1).

The conditions for reverse transcription may be the same as the conditions described in, for example, sections 2-2 to 2-4.

The reverse transcription reaction composition for preparing a reverse transcription reaction product for use in next-generation sequencing may contain components as described in sections 2-1, 2-2, and 2-3 (in particular, components necessary for reverse transcription) in addition to the above nucleic acid polymer.

The reverse transcription reaction composition for preparing a reverse transcription reaction product for use in next-generation sequencing may be in the form of a mixture of components or in the form of a kit that contains components separately.

### Examples

The present invention is described below in more detail with reference to Examples. However, the present invention is not limited to these Examples.

### Example 1: Suppression of Synthesis of DNA Derived from rRNA by Inosinic Acid Polymer in RT-PCR Method (Purified RNA: 10 pg)

In this Example, the following test was performed in order to examine the amount of ribosomal RNA-derived DNA synthesized with an inosinic acid polymer in an RT-PCR method.

A polyinosinic acid potassium salt (a potassium salt of an inosinic acid homopolymer (which is also referred to as "poly I")) was added as an inosinic acid polymer to a sample solution, and single-stranded cDNA was synthesized by a reverse transcription reaction. Thereafter, the amounts of ribosomal RNA-derived DNA synthesized with and without the polyinosinic acid potassium salt were compared according to a quantitative polymerase chain reaction (qPCR). Specifically, the following method was used.

The nucleic acid fragment sample used in this Example was RNA (10 pg) purified from NIH3T3 cells using an RNeasy Mini Kit (Qiagen).

Table 1 shows the components contained in the sample solution used in this Example for the reverse transcription reaction of the RNA (10 pg) and their final concentrations in the sample solution.

**Table 1**

| Final concentration | Component |
|---|---|
| 100 mM | Tris-HCl (pH of 8.0) |
| 10 mM | Magnesium chloride |
| 50 mM | Potassium chloride |
| 1 mM | dATP |
| 1 mM | dTTP |
| 1 mM | dGTP |
| 1 mM | dCTP |
| 0.4 µM | Oligo(dT)₁₈ primer |
| 4 µM | Random hexamer primer |
| 1 U/µl | Reverse transcriptase (ReverTra Ace produced by Toyobo Co., Ltd.) |
| 0.5 U/µl | RNase inhibitor (RNase Inhibitor produced by Toyobo Co., Ltd.) |
| 0 or 5 ng/µl | Polyinosinic acid potassium salt* (enzymatically synthesized product produced by Sigma-Aldrich) |

| | |
|---|---|
| *: Analysis of the base length distribution of the polyinosinic acid potassium salt with a Microchip Electrophoresis System (produced by Shimadzu Corporation) using a DNA-12000 reagent kit (produced by Shimadzu Corporation) found that as shown in Fig. 1, the polyinosinic acid potassium salt exhibits a peak at a length of about 450 bases and contains polyinosinic acid of about 150 to 8000 bases in length (reference value). | |

The reverse transcription reaction was performed using a sample solution obtained by adding the purified RNA to the composition described above. This reaction was performed at 37°C for 10 minutes, and then at 95°C for 5 minutes to synthesize single-stranded cDNA.

The amounts of DNA were then compared according to the following quantitative polymerase chain reaction (qPCR).

Each reverse transcription reaction liquid was diluted with nuclease-free water (Qiagen), and a 1/25 amount was used for a qPCR reaction. The qPCR was performed using StepOnePlus (Life Technologies) under the following conditions.

A qPCR reaction solution (20 µl (THUNDERBIRD (trademark) SYBR qPCR Mix (Toyobo Co., Ltd.), 6 pmol of forward primer, 6 pmol of reverse primer, 2 µl of diluted reverse transcription reaction liquid, and nuclease-free water) was treated at 95°C for 1 minute for enzyme activation, followed by 40 cycles of denaturation at 95°C for 15 seconds and an extension reaction at 60°C for 1 minute.

Melting curve analysis was performed at 95°C for 15 seconds, at 60°C for 15 seconds, and at 95°C for 15 seconds.

The target genes used were β-actin and Hprt-1, which are messenger RNA (mRNA), Neat-1, which is long non-coding RNA (lncRNA), and Rn18s, which is 18S ribosomal RNA.

The primers for each gene are as follows. β-actin (mRNA)
forward primer: CAGCTGAGAGGGAAATCGTG (SEQ ID NO: 1); reverse primer: CGTTGCCAATAGTGATGACC (SEQ ID NO: 2)
Neat-1 (lncRNA)
forward primer: GATCGGGACCCCAGTGACCT (SEQ ID NO: 3); reverse primer: AGCTTTCCCCAACACCCACA (SEQ ID NO: 4)
Hprt-1 (mRNA)
forward primer: TCAGTCAACGGGGGACATAAA (SEQ ID NO: 5); reverse primer: GGGGCTGTACTGCTTAACCAG (SEQ ID NO: 6)
Rn18s (ribosomal RNA)
forward primer: CTCAACACGGGAAACCTCAC (SEQ ID NO: 7); reverse primer: CGCTCCACCAACTAAGAACG (SEQ ID NO: 8)

Table 2 shows the results.

**Table 2**

| | Ct value | | | | Average Ct value | | ΔCt |
|---|---|---|---|---|---|---|---|
| Target gene | Poly I (no addition) | | Poly I (5 ng/µl) | | Poly I (no addition) | Poly I (5 ng/µl) | |
| β-actin (mRNA) | 24.9 | 24.6 | 25.3 | 25.2 | 24.7 | 25.3 | 0.6 |
| Neat-1 (lncRNA) | 28.6 | 28.1 | 29.4 | 29.7 | 28.4 | 29.6 | 1.2 |
| Hprt-1 (mRNA) | 31.7 | 29.8 | 31.2 | 30.6 | 30.7 | 30.9 | 0.2 |
| Rn18s (ribosomal RNA) | 18.6 | 17.8 | 22.6 | 22.3 | 18.2 | 22.4 | 4.2 |

ΔCt is a value obtained by subtracting the average Ct value under the condition without the polyinosinic acid potassium salt added from the average Ct value under the condition with the polyinosinic acid potassium salt added.

The difference between the Ct values with and without the polyinosinic acid potassium salt in Rn18s, which is ribosomal RNA, was larger than the difference between the Ct values with and without the polyinosinic acid potassium salt in β-actin and Hprt-1, which are mRNA, and Neat-1, which is lncRNA, (the difference between the amounts of mRNA- or lncRNA-derived DNA synthesized). This confirmed that by adding the polyinosinic acid potassium salt, the Ct value was delayed (increased) in Rn18s, compared with the other RNA, and the amount of ribosomal RNA-derived DNA synthesized was reduced.

Accordingly, it was confirmed that the synthesis of DNA derived from ribosomal RNA, which is usually undesired in reverse transcription, was suppressed by the inosinic acid polymer.

### Example 2: Suppression of Synthesis of DNA Derived from Ribosomal RNA by Inosinic Acid Polymer in RT-RamDA method (Purified RNA: 10 pg)

In this Example, the following test was performed in order to examine the amount of ribosomal RNA-derived DNA synthesized with an inosinic acid polymer in an RT-RamDA method.

A polyinosinic acid potassium salt was added to a sample solution, single-stranded cDNA was synthesized by an RT-RamDA method, and double-stranded cDNA was generated using the Klenow fragment. Thereafter, a library was prepared using *Nextera* (Illumina, Inc.), and next-generation sequencing (NGS) was performed with MiSeq (Illumina, Inc.) using a MiSeq Reagent Kit v3 (150 cycles) (Illumina, Inc.). The procedure was in accordance with the instruction manual. Analysis was performed with an Illumina BaseSpace TopHat (Illumina, Inc.) to calculate the percentage of ribosomal RNA in the reads. The procedure was in accordance with the instruction manual.

Specifically, the following method was used.

The nucleic acid fragment sample used in this Example was RNA (10 pg) purified from NIH3T3 cells using an RNeasy Mini Kit (Qiagen).

Table 3 shows the components contained in the sample solution used in this Example for subjecting the RNA (10 pg) to the RT-RamDA method and their final concentrations in the sample solution. The NSR primer (hexamer) was synthesized through the Sigma-Aldrich *custom oligo* service and mixed for use so as to have the primer composition disclosed in Ozsolak et al., Digital transcriptome profiling from attomole-level RNA samples, Genome Research, Vol. 20, 2010, pp. 519-525.

**Table 3**

| Final concentration | Component |
|---|---|
| 100 mM | Tris-HCl (pH of 8.0) |
| 10 mM | Magnesium chloride |
| 50 mM | Potassium chloride |
| 1 mM | dATP |
| 1 mM | dTTP |
| 1 mM | dGTP |
| 1 mM | dCTP |
| 0.4 µM | Oligo(dT)₁₈ primer |
| 4 µM | NSR primer (hexamer) |
| 1 U/µl | Reverse transcriptase (ReverTra Ace produced by Toyobo Co., Ltd.) |
| 0.5 U/µl | RNase inhibitor (RNase Inhibitor produced by Toyobo Co., Ltd.) |
| 0.2 U/µl | DNase I (DNase I produced by Thermo Fisher Scientific) |
| 50 ng/µl | T4 gene 32 protein |
| 0, 1, or 3 ng/µl | Polyinosinic acid potassium salt used in Example 1 |

The RT-RamDA method was performed using a sample solution obtained by adding the purified RNA to the composition described above. In this method, the reaction was performed at 25°C for 10 minutes, at 30°C for 10 minutes, at 37°C for 30 minutes, at 50°C for 5 minutes, and at 85°C for 5 minutes. Table 4 shows the results.

**Table 4**

| | Number of reads | Percentage total aligned | Percentage abundant | Percentage abundant/ percentage total aligned |
|---|---|---|---|---|
| 1 ng/µl polyinosinic acid potassium salt | 713,069 | 83.65% | 23.86% | 28.52% |
| 3 ng/µl polyinosinic acid potassium salt | 878,539 | 74.96% | 16.78% | 22.39% |
| No polyinosinic acid potassium salt added | 943,238 | 88.95% | 41.68% | 46.86% |

"Number of reads" in Table 4 is the total number of reads obtained. "Percentage total aligned" indicates the percentage of reads that matched a reference genome (UCSC mm10) including ribosomal RNA in the total reads, and "Percentage abundant" indicates the percentage of ribosomal RNA. "Percentage abundant/percentage total aligned" indicates the percentage of ribosomal RNA in the reads that matched the reference genome.

The percentage abundant/percentage total aligned was lower in the conditions with the polyinosinic acid potassium salt added than in the condition without the polyinosinic acid potassium salt added, indicating that the percentage of ribosomal RNA was reduced.

Accordingly, it was confirmed that the synthesis of DNA derived from ribosomal RNA in the RT-RamDA method was suppressed by the inosinic acid polymer.

### Example 3: Suppression of Synthesis of DNA Derived from Ribosomal RNA by Inosinic Acid Polymer in RT-RamDA Method (NIH3T3 cells)

In this Example, the following test was performed in order to examine the amount of ribosomal RNA-derived DNA synthesized with an inosinic acid polymer in a RamDA reaction.

A polyinosinic acid potassium salt was added to a sample solution, single-stranded cDNA was synthesized by an RT-RamDA method, and double-stranded cDNA was generated using the Klenow fragment. Thereafter, a library was prepared using *Nextera* (Illumina, Inc.), and next-generation sequencing (NGS) was performed with MiSeq (Illumina, Inc.) using a MiSeq Reagent Kit v3 (150 cycles) (Illumina, Inc.). The procedure was in accordance with the instruction manual. Analysis was performed with an Illumina BaseSpace TopHat (Illumina, Inc.) to calculate the percentage of ribosomal RNA in the reads. The procedure was in accordance with the instruction manual.

Specifically, the following method was used.

The cell sample used in this Example was prepared so as to have the following composition.

NIH3T3 cells were reacted at 37°C for 2 minutes using a trypsin solution (Nacalai Tesque, Inc.) to be dissociated into single cells. After the dissociation, the reaction was immediately stopped by replacement with PBS(-). Cells that were negative for PI, which is a dead cell fluorescence marker dye, were defined as a living cell fraction using a FACSMelody Cell Sorter (BD). From this fraction, 40 cells were placed in 3 µl of the following lysis buffer. Immediately after that, the cells were centrifuged and stored at -80°C. When the cells were used for a reverse transcription reaction, 117 µl of the lysis buffer was added after the cells were thawed, and 3 µl of the 40-fold-diluted cell lysate was used as a 1-cell lysate.

### Lysis Buffer

0.1 mg/ml protease K
0.5% Triton X-100
0 or 5 ng/ul polyinosinic acid potassium salt used in Example 1
1 U/ul RNase inhibitor (RNase Inhibitor produced by Toyobo Co., Ltd.)

Table 5 shows the components contained in the sample solution used in this Example for subjecting the 1-cell lysate to the RT-RamDA method and their final concentrations in the sample solution.

**Table 5**

| Final concentration | Component |
|---|---|
| 100 mM | Tris-HCl (pH of 8.0) |
| 10 mM | Magnesium chloride |
| 50 mM | Potassium chloride |
| 1 mM | dATP |
| 1 mM | dTTP |
| 1 mM | dGTP |
| 1 mM | dCTP |
| 0.4 µM | Oligo(dT)₁₈ primer |
| 4 µM | NSR primer (hexamer) used in Example 2 |
| 1 U/µl | Reverse transcriptase (ReverTra Ace produced by Toyobo Co., Ltd.) |
| 0.5 U/µl | RNase inhibitor (RNase Inhibitor produced by Toyobo Co., Ltd.) |
| 0.2 U/µl | DNase I (DNase I produced by Thermo Fisher Scientific) |
| 50 ng/µl | T4 gene 32 protein |

3 µl of the 1-cell lysate was heat-treated at 70°C for 2 minutes and added to 6 µl of the sample solution having the composition described above, and the RT-RamDA method was performed (the final concentration of the polyinosinic acid potassium salt in the reaction liquid was 0 or 1.67 ng/µl). In this method, the reaction was performed at 25°C for 10 minutes, at 30°C for 10 minutes, at 37°C for 30 minutes, at 50°C for 5 minutes, and at 85°C for 5 minutes.

Table 6 shows the results.

**Table 6**

| | Number of reads | Percentage total aligned | Percentage abundant | Percentage abundant/ percentage total aligned |
|---|---|---|---|---|
| No polyinosinic acid potassium salt added | 852993 | 89.21% | 38.36% | 43.00% |
| 5 ng/µl polyinosinic acid potassium salt | 712267 | 87.71% | 24.28% | 27.68% |

The percentage abundant/percentage total aligned was lower in the condition with the polyinosinic acid potassium salt added than in the condition without the polyinosinic acid potassium salt added, indicating that the percentage of ribosomal RNA was reduced.

Accordingly, it was confirmed that by incorporating the polyinosinic acid potassium salt into the cell lysate, the polyinosinic acid potassium salt was consequently added to the sample solution in the RT-RamDA method, and the synthesis of DNA derived from ribosomal RNA was suppressed by the polyinosinic acid potassium salt.

### Example 4: Suppression of Synthesis of DNA Derived from Ribosomal RNA by Inosinic Acid Polymer, Deoxyguanylic Acid Polymer,_ Adenylic Acid Polymer, and Deoxythymidylic Acid Polymer in RT-PCR Method (NIH3T3 cells)

In this Example, the following test was performed in order to examine the amounts of ribosomal RNA-derived DNA synthesized with four nucleic acid polymers (a polyinosinic acid potassium salt as an inosinic acid polymer, polydeoxyguanylic acid as a deoxyguanylic acid polymer, a polyadenylic acid potassium salt as an adenylic acid polymer, and polydeoxythymidylic acid as a deoxythymidylic acid polymer) in an RT-PCR method.

Each nucleic acid polymer was individually added to a cell lysate, and single-stranded cDNA was synthesized by a reverse transcription reaction. Thereafter, the amounts of DNA synthesized were compared according to a quantitative polymerase chain reaction (qPCR). Specifically, the following method was used.

The cell sample used in this Example was prepared so as to have the following composition.

NIH3T3 cells were reacted at 37°C for 2 minutes using a trypsin solution (Nacalai Tesque, Inc.) to be dissociated into single cells. After the dissociation, the reaction was immediately stopped by replacement with PBS(-). Cells that were negative for PI, which is a dead cell fluorescence marker dye, were defined as a living cell fraction using a FACSMelody Cell Sorter (BD). From this fraction, 10 cells were placed in 3 µl of the following lysis buffer. Immediately after that, the cells were centrifuged and stored at -80°C. When the cells were used for a reverse transcription reaction, the cells were heat-treated after thawing, and 3 µl of the cell lysate was used as a 10-cell lysate.

### Lysis Buffer

0.1 mg/ml protease K
0.1% *Nonidet*-40
1 U/µl RNase inhibitor (RNase Inhibitor produced by Toyobo Co., Ltd.)

Table 7 shows the components contained in the sample solution used in this Example for the reverse transcription reaction of the cell lysate and their final concentrations in the sample solution.

**Table 7**

| Final concentration | Component |
|---|---|
| 100 mM | Tris-HCl (pH of 8.0) |
| 10 mM | Magnesium chloride |
| 50 mM | Potassium chloride |
| 1 mM | dATP |
| 1 mM | dTTP |
| 1 mM | dGTP |
| 1 mM | dCTP |
| 0.4 µM | Oligo (dT)₁₈ primer |
| 4 µM | NSR primer (hexamer) used in Example 2 |
| 1 U/µl | Reverse transcriptase (ReverTra Ace produced by Toyobo Co., Ltd.) |
| 0.5 U/µl | RNase inhibitor (RNase Inhibitor produced by Toyobo Co., Ltd.) |
| 0, 0.2, 2, or 20 ng/µl | Polyinosinic acid potassium salt used in Example 1 |
| | Polydeoxyguanylic acid (about 70 bases in length; chemically synthesized product produced by Hokkaido System Science Co., Ltd.) |
| | Polyadenylic acid potassium salt* (P9403, enzymatically synthesized product produced by Sigma-Aldrich), or |
| | Polydeoxythymidylic acid (about 70 bases in length; chemically synthesized product produced by Hokkaido System Science Co., Ltd.) |

| | |
|---|---|
| *: Analysis of the base length distribution of the polyadenylic acid potassium salt with a Microchip Electrophoresis System (produced by Shimadzu Corporation) using a DNA-12000 reagent kit (produced by Shimadzu Corporation) found that as shown in Fig. 2, the polyadenylic acid potassium salt exhibits a peak at a length of about 300 bases and contains polyadenylic acid of about 100 to 5000 bases in length (reference value). | |

3 µl of the cell lysate of 10 cells was heat-treated at 70°C for 2 minutes and added to the composition described above, and the reverse transcription reaction was performed. This reaction was performed at 37°C for 10 minutes and then at 95°C for 5 minutes, thereby synthesizing single-stranded cDNA.

Thereafter, the amounts of DNA amplified were compared according to the following quantitative polymerase chain reaction (qPCR).

Each reverse transcription reaction liquid was diluted with nuclease-free water (Qiagen), and a 1/25 amount was used for a qPCR reaction. The qPCR was performed using a StepOne Plus (Life Technologies) under the following conditions. A qPCR reaction solution (20 µl (THUNDERBIRD (trademark) SYBR qPCR Mix (Toyobo Co., Ltd.), 6 pmol of forward primer, 6 pmol of reverse primer, 2 µl of diluted reverse transcription reaction liquid, and nuclease-free water) was treated at 95°C for 1 minute for enzyme activation, followed by 40 cycles of denaturation at 95°C for 15 seconds and an extension reaction at 60°C for 1 minute. Melting curve analysis was performed at 95°C for 15 seconds, at 60°C for 15 seconds, and at 95°C for 15 seconds. The target genes used were β-actin (mRNA) and Rn18s (18S ribosomal RNA).

Table 8 shows the results.

**Table 8**

| | | - | Polyinosinic acid potassium salt | | | Polydeoxyguanylic acid | | | Polyadenylic acid potassium salt | | | Polydeoxythymidylic acid | |
|---|---|---|---|---|---|---|---|---|---|---|---|---|---|
| | Amount added | No addition | 0.2 ng /µl | 2ng /µl | 20 ng /µl | 0.2 ng /µl | 2ng /µl | 20 ng /µl | 0.2 ng /µl | 2ng /µl | 20 ng /µl | 0.2 ng /µl | 2ng /µl |
| Ct value | β-actin | 26.6 | 27.4 | 26.5 | 27.5 | 25.9 | 26.5 | 27.5 | 26.2 | 26.5 | 26.9 | 26.3 | 26.5 |
| | Rn18s | 19.7 | 21.8 | 23.2 | 27.2 | 21.6 | 23.6 | 25.8 | 20.8 | 22.2 | 23.5 | 21.3 | 21.8 |
| ΔCt value | β-actin | - | 0.7 | -0.2 | 0.9 | -0.7 | -0.1 | 0.9 | -0.5 | -0.2 | 0.2 | -0.3 | -0.1 |
| | Rn18s | - | 2.1 | 3.5 | 7.5 | 1.9 | 3.8 | 6.0 | 1.1 | 2.5 | 3.8 | 1.6 | 2.1 |

ΔCt is a value obtained by subtracting the average Ct value under the condition without each nucleic acid polymer added from the average Ct value under the condition with the nucleic acid polymer added.

The difference between the Ct values with and without each nucleic acid polymer in Rn18s, which is ribosomal RNA, was larger than the difference between the Ct values with and without the nucleic acid polymer in β-actin, which is mRNA (the difference between the amounts of mRNA-derived DNA synthesized). This result revealed that by adding the polyinosinic acid potassium salt or polydeoxyguanylic acid, the Ct value was greatly delayed (increased) in Rn18s, compared with the other RNA, and the synthesis of DNA derived from rRNA was suppressed. It was also confirmed that the synthesis of DNA derived from rRNA was further suppressed by increasing the amount of the polyinosinic acid potassium salt or polydeoxyguanylic acid added. Also by using the polyadenylic acid potassium salt, the Ct value was delayed (increased), and the synthesis of DNA derived from rRNA was suppressed, although the degree was somewhat lower than in the case of using the polyinosinic acid potassium salt or polydeoxyguanylic acid. In the case of using polydeoxythymidylic acid, it was confirmed that although the delay in the Ct value was smaller than that in the other nucleic acid polymers, the Ct value was slightly delayed.

Accordingly, it was confirmed that the synthesis of DNA derived from ribosomal RNA, which is usually undesired in reverse transcription, was suppressed by adding each nucleic acid polymer.

## Claims

1. A method for synthesizing DNA by reverse transcription of template RNA, the method comprising suppressing synthesis of DNA derived from ribosomal RNA using at least one nucleic acid polymer selected from the group consisting of inosinic acid polymers, cytidylic acid polymers, guanylic acid polymers, adenylic acid polymers, thymidylic acid polymers, uridylic acid polymers, deoxyinosinic acid polymers, deoxycytidylic acid polymers, deoxyguanylic acid polymers, deoxyadenylic acid polymers, deoxythymidylic acid polymers, and deoxyuridylic acid polymers.

2. The method according to claim 1, wherein the nucleic acid polymer is at least one homopolymer selected from the group consisting of polyinosinic acid, polycytidylic acid, polyguanylic acid, polyadenylic acid, polythymidylic acid, polyuridylic acid, polydeoxyinosinic acid, polydeoxycytidylic acid, polydeoxyguanylic acid, polydeoxyadenylic acid, polydeoxythymidylic acid, polydeoxyuridylic acid, and salts thereof.

3. The method according to claim 1 or 2, wherein the nucleic acid polymer is at least one homopolymer selected from the group consisting of polyinosinic acid, polycytidylic acid, polyguanylic acid, polydeoxyinosinic acid, polydeoxycytidylic acid, polydeoxyguanylic acid, and salts thereof.

4. The method according to any one of claims 1 to 3, wherein the nucleic acid polymer is at least one homopolymer selected from the group consisting of polyinosinic acid, polydeoxyinosinic acid, and salts thereof.

5. The method according to any one of claims 1 to 4, wherein the nucleic acid polymer has a total length of 30 to 10000 bases.

6. The method according to any one of claims 1 to 5, wherein the reverse transcription is performed by using a random primer.

7. The method according to claim 6, wherein the random primer has a base sequence that is not completely complementary to the base sequence of the ribosomal RNA.

8. The method according to any one of claims 1 to 7, wherein the reverse transcription is performed in an RT-PCR method or RT-RamDA method.

9. A composition for suppressing reverse transcription of ribosomal RNA, the composition comprising at least one nucleic acid polymer selected from the group consisting of inosinic acid polymers, cytidylic acid polymers, guanylic acid polymers, adenylic acid polymers, thymidylic acid polymers, uridylic acid polymers, deoxyinosinic acid polymers, deoxycytidylic acid polymers, deoxyguanylic acid polymers, deoxyadenylic acid polymers, deoxythymidylic acid polymers, and deoxyuridylic acid polymers.

10. A reverse transcription reaction composition for preparing a reverse transcription reaction product for use in next-generation sequencing, the reverse transcription reaction composition comprising at least one nucleic acid polymer selected from the group consisting of inosinic acid polymers, cytidylic acid polymers, guanylic acid polymers, adenylic acid polymers, thymidylic acid polymers, uridylic acid polymers, deoxyinosinic acid polymers, deoxycytidylic acid polymers, deoxyguanylic acid polymers, deoxyadenylic acid polymers, deoxythymidylic acid polymers, and deoxyuridylic acid polymers.

11. The composition according to claim 9 or 10, wherein the reverse transcription is performed in an RT-PCR method or RT-RamDA method.
